Europäisches Patentamt

European Patent Office

Office européen des brevets

(19)

(11) EP 0 733 616 A1

(12) **EUROPÄISCHE PATENTANMELDUNG**

(43) Veröffentlichungstag:
25.09.1996 Patentblatt 1996/39

(51) Int. Cl.$^6$: **C07C 59/06**, C07C 51/43

(21) Anmeldenummer: 96104128.2

(22) Anmeldetag: 15.03.1996

(84) Benannte Vertragsstaaten:
BE CH DE ES FR GB IT LI NL

(30) Priorität: 21.03.1995 DE 19510156

(71) Anmelder: HOECHST AKTIENGESELLSCHAFT
65929 Frankfurt am Main (DE)

(72) Erfinder:
• Forbert, Rainald, Dr.
  65439 Flörsheim (DE)
• Rittner, Siegbert, Dr.
  64546 Mörfelden-Walldorf (DE)
• Hermann, Kurt, Ing.
  65510 Idstein (DE)
• Ebmeyer, Frank, Dr.
  86153 Augsburg (DE)

(54) **Verfahren zur Herstellung kristalliner Glykolsäure**

(57)     Die Erfindung betrifft ein Verfahren zur Herstellung kristalliner Glykolsäure aus einer wäßrigen Glykolsäurelösung. Die Erfindung zeichnet sich dadurch aus, daß man der wäßrigen Glykolsäurelösung bei erhöhter Temperatur das Wasser entzieht und daß man die anfallende Schmelze mit Kristallisationskeimen vermischt und abkühlt. Eine besondere Variante dieses Verfahrens ist dadurch gekennzeichnet, daß man der wäßrigen Glykolsäurelösung das Wasser innerhalb einer Zeit von 5 Minuten entzieht. Die Vorteile des Verfahrens sind darin zu sehen, daß auf umweltfreundliche Weise großtechnisch kristalline Glykolsäure gewonnen werden kann, die zudem arm an Verunreinigungen, insbesondere an Polyglykoliden ist.

EP 0 733 616 A1

## Beschreibung

Die vorliegende Erfindung betrifft ein Verfahren zur Herstellung kristalliner Glykolsäure aus einer wässerigen Glykolsäurelösung.

Glykolsäure ist ein wichtiges End- und Zwischenprodukt für vielfältige Anwendungen. Sie wird z.B. in großem Umfang in der Textil-, Leder- und Pelzverarbeitung, in der Kosmetik-Industrie, sowie bei der Metallreinigung, der Metallplattierung und der Desinfektion von Molkereigeräten eingesetzt. Ein weiteres Einsatzgebiet ist die Verminderung der Kesselsteinbildung in Wärmeaustauschern durch Einstellung eines definierten pH-Wertes mit Hilfe von Glykolsäure. Des weiteren findet sie Verwendung bei der Chelatisierung von Calcium und Eisenionen. Steigende Bedeutung gewinnt Glykolsäure auch als Synthesebaustein z.B. für Arzneimittel, Pflanzenschutzmittel und zur Gewinnung von Estern als Weichmacher für Polymere.

Aus dem Stand der Technik ist ein Verfahren zur Herstellung von kristalliner Glykolsäure aus einer wässerigen Glykolsäurelösung bekannt. Entsprechend den Angaben in Ullmanns Enzyklopädie der technischen Chemie, 3. Aufl., Bd. 13 (1962), S. 77 kann kristalline Glykolsäure im Labormaßstab hergestellt werden, indem man Glykolsäuremethylester mit Wasser unter Rückfluß kocht, danach das Methanol abdestilliert und die entstandene wässerige Glykolsäurelösung bei einer Temperatur $\leq$ 40°C im Vakuum eindampft, anschließend abkühlt und die kristallisierte Glykolsäure absaugt.

Die großtechnische Herstellung gut konfektionierbarer fester Glykolsäure bereitet jedoch große Schwierigkeiten. Dies liegt daran, daß sich bei der Eindampfung wässeriger Glykolsäurelösungen im Sinne der nachstehenden Reaktionsgleichung

$$\text{n } HOCH_2COOH \Leftrightarrow H(OCH_2CO)_nOH + (n\text{-}1) \text{ } H_2O$$

Polyglykolide bilden können, die besonders dann störend sind, wenn die Glykolsäure z.B. als Synthesebaustein in wasserfreien Medien eingesetzt wird. Wegen dieser Problematik ist heute Glykolsäure im großtechnischen Maßstab nur als wässerig-konzentrierte Lösung im Handel anzutreffen. Die in kleineren Mengen in fester Form angebotene Glykolsäure wird überwiegend durch Kristallisation aus organischen Lösungsmitteln, wie z.B. Aceton, gewonnen. Diese Verfahren sind jedoch wegen der erforderlichen Aufarbeitung des Lösungsmittels großtechnisch nicht nur teuer, sondern erfordern auch kostspielige Sicherheits- und Umweltschutz-Maßnahmen, wie sie beim Arbeiten mit großen Lösungsmittelmengen heute unerläßlich sind.

Der Erfindung liegt daher die Aufgabe zugrunde, ein Verfahren zur Herstellung kristalliner Glykolsäure aus wässeriger Glykolsäurelösung zu entwickeln, das umwelttechnisch vorteilhaft ist und großtechnisch ausgeübt werden kann.

Diese Aufgabe wird dadurch gelöst, daß man der wässerigen Glykolsäurelösung bei erhöhter Temperatur das Wasser entzieht und daß man die anfallende Schmelze mit Kristallisationskeimen vermischt und abkühlt.

Gegenstand der Erfindung ist somit ein Verfahren zur Herstellung kristalliner Glykolsäure aus einer wässerigen Glykolsäurelösung, dadurch gekennzeichnet, daß man der wässerigen Glykolsäurelösung bei erhöhter Temperatur das Wasser entzieht und daß man die anfallende Schmelze mit Kristallisationskeimen vermischt und abkühlt. "Erhöhte Temperatur" bedeutet hier größer als 20°C.

Eine besondere Ausführungsform ist dadurch gekennzeichnet, daß man das Wasser bei einer Temperatur aus dem Bereich von 30 bis 95°C, insbesondere von 40 bis 85°C entzieht.

Eine weitere Ausführungsform entsteht, wenn man das Wasser im Vakuum bei einem Absolutdruck aus dem Bereich von 1 bis 20 mbar, insbesondere von 5 bis 10 mbar entzieht.

Ebenfalls ist es vorteilhaft, wenn man der wässerigen Glykolsäurelösung das Wasser innerhalb einer Zeit zwischen 0 und 5 Minuten, insbesondere von 0,1 bis 3 Minuten und vorzugsweise 0,5 bis 2 Minuten entzieht.

Weiter ist es vorteilhaft, wenn man die Schmelze auf eine Temperatur zwischen 0 und 30°C, besonders zwischen 10 und 20°C abkühlt.

Eine weitere Ausführungsform der Erfindung ist dadurch gekennzeichnet, daß die Entziehung des Wassers oder die Vermischung oder die Abkühlung unter Inertgasatmosphäre durchgeführt wird.

Weitere Ausführungsformen ergeben sich aus den Ansprüchen 7 bis 14.

Als Ausgangsprodukt für das erfindungsgemäße Verfahren sind grundsätzlich alle wässerigen Glykolsäurelösungen aus den bekannten Synthesewegen geeignet. Bevorzugt sind dabei wässerige Lösungen, die aus hochgereinigter Monochloressigsäure (> 99 Gew.-%) über das alkalische Hydrolyseverfahren mit anschließender Ansäuerung gewonnen wurden. Bevorzugte handelsübliche Glykolsäurelösungen enthalten etwa 20 bis 57 oder 70 Gew.-% Glykolsäure. Als Kristallisationskeime eignen sich vorzugsweise feste Glykolsäureteilchen.

Das erfindungsgemäße Verfahren ist auch mit einzelnen oder mehreren Merkmalen aus den verschiedenen Ausführungsformen beliebig kombinierbar.

Die Erfindung zeigt den überraschenden Effekt, daß eine gut kristalline Glykolsäure auch großtechnisch direkt aus wässerigen Lösungen gewonnen werden kann, wenn eine Kombination aus Entwässerung und anschließender Konfektionierung, bestehend aus der Vermischung mit Kristallisationskeimen und Abkühlung, durchgeführt wird. Dabei muß die Heiztemperatur nicht zwangsläufig unter 40°C liegen, wie in Ullmanns Enzyklopädie der technischen Chemie, 3. Aufl., Bd. 13 (1962), S. 77 angegeben, sondern kann auch darüber liegen, wenn erfindungsgemäß verfahren wird.

Die Vorteile des erfindungsgemäßen Verfahrens sind darin zu sehen, daß auf umweltfreundliche Weise großtechnisch kristalline Glykolsäure gewonnen werden kann, die zudem arm an Verunreinigungen, insbesondere an Polyglykoliden ist.

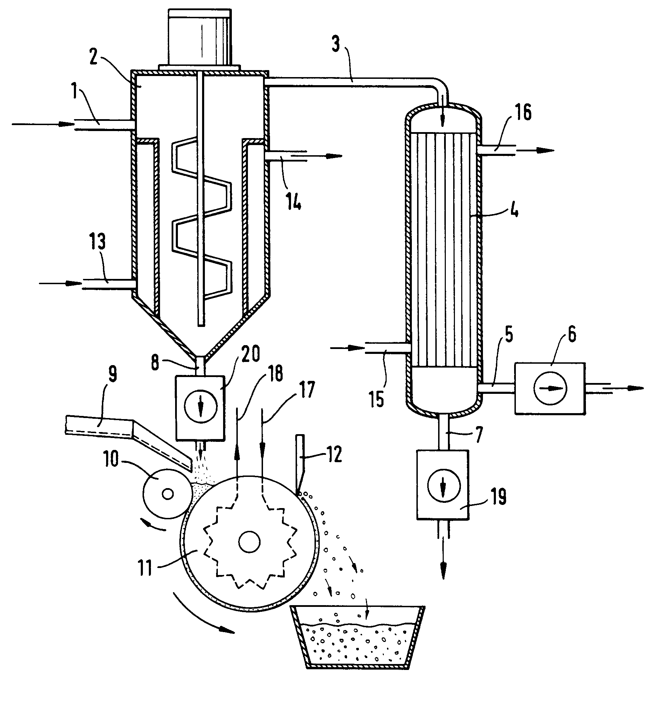

Das erfindungsgemäße Verfahren wird im folgenden anhand eines in der einzigen Figur beispielhaft dargestellten Fließbildes näher erläutert.

Eine wässerige Glykolsäurelösung wird über eine Leitung 1 einem ein- oder mehrstufigen Verdampfer, vorzugsweise einen senkrechtstehenden, wasser- oder ölbeheizten Dünnschichtverdampfer 2 oben zugeführt. Die wässerige Glykolsäurelösung fließt im Dünnschichtverdampfer 2 von oben nach unten, wobei das Wasser verdampft und eine viskose Schmelze entsteht. Die entstehenden Dämpfe werden über eine Leitung 3 abgesaugt und in einem Kondensator 4 kondensiert. Die nicht kondensierten Dämpfe werden weiter über eine Leitung 5 mittels einer Vakuumanlage 6 abgesaugt, wodurch im Dünnschichtverdampfer 2 und im Kondensator 4 ein Vakuum bis zu einem Absolutdruck von 1 mbar eingestellt werden kann. Das Kondensat kann den Kondensator 4 durch einen Ausgang 7 verlassen.

Die Schmelze wird dem Dünnschichtverdampfer 2 an einen Ausgang 8 entnommen und zusammen mit Kristallisationskeimen, die über eine Dosiervorrichtung, vorzugsweise eine Vibrationsrinne 9 zugegeben werden, in eine Misch- und Kühlvorrichtung gebracht. Die Misch- und Kühlvorrichtung besteht vorzugsweise aus zwei gegenläufig rotierenden Walzen, einer kleineren Walze 10 und einer größeren, kühlbaren Walze 11, die in einem einstellbaren Abstand so zueinander ortsfest angeordnet sind, daß ein enger Spalt entsteht.

Durch diesen Spalt werden Schmelze und Kristallisationskeime hindurchgeführt und dabei gleichzeitig innig verknetet und gekühlt.
Die viskose Schmelze kristallisiert auf der Oberfläche der gekühlten, rotierenden Walze 11 zu fester Glykolsäure, wird mitgenommen und nach etwa einer dreiviertel Umdrehung mit Hilfe eines ortsfest über der Oberfläche der Walze 11 angeordneten Messers 12 von der Walzenoberfläche entfernt.
Die Zahlen 13 und 14 bezeichnen einen Eingang und einen Ausgang für ein Heizmedium, die Zahlen 15 und 16 einen Eingang und einen Ausgang für ein Kühlmedium.

Nachstehend wird das erfindungsgemäße Verfahren anhand einiger Ausführungsbeispiele weiter beschrieben, ohne dadurch beschränkt zu werden.

Beispiel 1

8,5 kg/h einer 57 %igen (Gew.-%) wässerigen Glykolsäurelösung mit 13 % Kochsalz-Gehalt wurden in einem Dünnschichtverdampfer mit einer Heizfläche von 0,125 m$^2$ bei einem Druck von 10 mbar und unter Beheizung mit 76°C eingedampft. Die milchig-weiße und geruchlose Schmelze wurde mit Impfkristallen innig verknetet und sodann einer Kühlwalze von 0,6 m$^2$ Fläche und einer Umdrehungszahl von 3 U/min zugeführt. Die Kühltemperatur der Walze betrug dabei 15°C. Das in Schuppenform anfallende weiße, geruchlose, kristalline Produkt hatte die folgende Zusammensetzung: 75 % Glykolsäure, 18 % Kochsalz, 5,8 % Polyglykolide und 1,2 % Wasser. Das Produkt ließ sich gut und verklebungsfrei handhaben. Die Ausbeute an Glykolsäure (einschließlich Polyglykolide) betrug über 99 %.

Beispiel 2

9 kg/h einer 65 %igen, kochsalzfreien, wässerigen Glykolsäurelösung wurden im gleichen Dünnschichtverdampfer wie im Beispiel 1 bei einem Druck von 10 mbar und unter Beheizung mit 80°C eingedampft. Die klare und geruchlose Schmelze wurde mit Impfkristallen innig verknetet und sodann derselben Kühlwalze wie in Beispiel 1 und unter den gleichen Betriebsbedingungen zugeführt. Das in Schuppenform anfallende farb- und geruchlose kristalline Produkt hatte die folgende Zusammensetzung: 94 % Glykolsäure, 4,5 % Polyglykolide und 1,5 % Wasser. Das Produkt ließ sich gut und verklebungsfrei handhaben. Die Ausbeute an Glykolsäure (einschließlich Polyglykolide) betrug über 99 %.

Beispiel 3

12 kg/h einer wie in Beispiel 1 beschriebenen wässerigen Glykolsäurelösung wurden im gleichen Dünnschichtverdampfer bei einem Druck von 20 mbar und unter Beheizung mit 85°C eingedampft. Die milchig weiße und geruchlose Schmelze wurde mit Impfkristallen innig verknetet und sodann derselben Kühlwalze wie in Beispiel 1 unter den gleichen Betriebsbedingungen zugeführt. Das in Schuppenform anfallende weiße geruchlose kristalline Produkt hatte die folgende Zusammensetzung: 73 % Glykolsäure, 18 % Kochsalz, 6,5 % Polyglykolide und 2,5 % Wasser. Das Produkt ließ sich gut und verklebungsfrei handhaben. Die Ausbeute an Glykolsäure (einschließlich Polyglykolide) betrug über 98 %.

Beispiel 4

4 kg/h einer wie in Beispiel 1 beschriebenen wässerigen Glykolsäurelösung wurden im gleichen Dünnschichtverdampfer bei einem Druck von 1 mbar und unter Beheizung mit 50°C eingedampft. Die milchig weiße und geruchlose Schmelze wurde mit Impfkristallen innig verknetet und sodann derselben Kühlwalze wie in Beispiel 1 unter den gleichen Betriebsbedingungen zugeführt. Das in Schuppenform anfallende weiße geruchlose kristalline Produkt hatte die folgende Zusammensetzung: 77 % Glykolsäure, 18 % Kochsalz, 4 % Polyglykolide und 1 % Wasser. Das Produkt ließ sich gut und verklebungsfrei handhaben. Die Ausbeute an Glykolsäure (einschließlich Polyglykolide) betrug über 99 %.

**Patentansprüche**

1. Verfahren zur Herstellung kristalliner Glykolsäure aus einer wässerigen Glykolsäurelösung, dadurch gekennzeichnet, daß man der wässerigen Glykolsäurelösung bei erhöhter Temperatur das Wasser entzieht und daß man die anfallende Schmelze mit Kristallisationskeimen vermischt und abkühlt.

2. Verfahren nach Anspruch 1, dadurch gekennzeichnet, daß man das Wasser bei einer Temperatur aus dem Bereich von 30 bis 95°C entzieht.

3. Verfahren nach Anspruch 1 oder 2, dadurch gekennzeichnet, daß man das Wasser im Vakuum bei einem Absolutdruck aus dem Bereich von 1 bis 20 mbar entzieht.

4. Verfahren nach einem oder mehreren der Ansprüche 1 bis 3, dadurch gekennzeichnet, daß man der wässerigen Glykolsäurelösung das Wasser innerhalb einer Zeit zwischen 0 und 5 Minuten entzieht.

5. Verfahren nach einem oder mehreren der Ansprüche 1 bis 4, dadurch gekennzeichnet, daß man die Schmelze auf eine Temperatur zwischen 0 und 30°C abkühlt.

6. Verfahren nach einem oder mehreren der Ansprüche 1 bis 5, dadurch gekennzeichnet, daß die Entziehung des Wassers oder die Vermischung oder die Abkühlung unter Inertgasatmosphäre durchgeführt wird.

7. Verfahren nach einem oder mehreren der Ansprüche 1 bis 6, dadurch gekennzeichnet, daß die Entziehung des Wassers mehrstufig erfolgt.

8. Verfahren nach einem oder mehreren der Ansprüche 1 bis 7, dadurch gekennzeichnet, daß der Gehalt an Glykolsäure in der eingesetzten wässerigen Lösung mindestens 20 % beträgt.

9. Verfahren nach einem oder mehreren der Ansprüche 1 bis 8, dadurch gekennzeichnet, daß die eingesetzte wässerige Glykolsäurelösung Glykolsäure enthält, die durch alkalische Hydrolyse von reinen Monochloressigsäuren gewonnen wurde.

10. Verfahren nach Anspruch 9, dadurch gekennzeichnet, daß die Reinheiten der zur alkalischen Hydrolyse eingesetzten Monochloressigsäure mindestens 99 % betragen.

11. Verfahren nach einem oder mehreren der Ansprüche 1 bis 10, dadurch gekennzeichnet, daß zur Entziehung des Wassers ein Dünnschichtverdampfer verwendet wird.

12. Verfahren nach einem oder mehreren der Ansprüche 1 bis 11, dadurch gekennzeichnet, daß die Vermischung mit Hilfe von Walzen erfolgt.

13. Verfahren nach einem oder mehreren der Ansprüche 1 bis 12, dadurch gekennzeichnet, daß die Abkühlung mit Hilfe von Kühlwalzen oder eines Kühlbandes erfolgt.

14. Verfahren nach einem oder mehreren der Ansprüche 1 bis 13, dadurch gekennzeichnet, daß die Kristallisationskeime feste Glykolsäureteilchen sind.

**Europäisches Patentamt**

# EUROPÄISCHER RECHERCHENBERICHT

Nummer der Anmeldung

EP 96 10 4128

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | US-A-2 341 258 (APPEL)<br><br>* Seite 1, Spalte 2, Zeile 10 - Zeile 30 *<br>* Seite 2, Spalte 1, Zeile 30 - Zeile 67 *<br>* Seite 2, Spalte 2, Zeile 62 - Seite 3, Spalte 1, Zeile 65 *<br>* Abbildung 1 *<br>* Ansprüche 1-8 *<br>--- | 1-8, 11-13 | C07C59/06<br>C07C51/43 |
| X | US-A-2 028 064 (GRETHER)<br>* Anspruch 8 *<br>--- | 9 | |
| A | DR.OTTO-ALBRECHT NEUMÜLLER: "RÖMPPS CHEMIE-LEXIKON, ACHTE AUFLAGE, BAND 3: H-L"<br>1984 , FRANCKH'SCHE VERLAGSHANDLUNG , STUTTGART XP002006557<br>STICHWORT "KRISTALLISATION"<br>* Seite 2244 - Seite 2246 *<br>----- | 1,14 | |
| | | | **RECHERCHIERTE SACHGEBIETE (Int.Cl.6)**<br><br>C07C |

Der vorliegende Recherchenbericht wurde für alle Patentansprüche erstellt

| Recherchenort | Abschlußdatum der Recherche | Prüfer |
|---|---|---|
| DEN HAAG | 27.Juni 1996 | Klag, M |

EPO FORM 1503 03.82 (P04C03)